# EUROPEAN PATENT SPECIFICATION

(11) **EP 1 079 752 B1**
(45) Date of publication and mention of the grant of the patent: **05.01.2005**
(21) Application number: 99914726.7
(22) Date of filing: 04.05.1999
(51) Int. Cl.: A61B 17/68

(54) **SYNTHESIS DEVICE FOR ORTHOPAEDIA AND TRAUMATOLOGY**
SYNTHESEVORRICHTUNG FÜR ORTHOPÄDIE UND TRAUMATOLOGIE
DISPOSITIF SYNTHETIQUE A USAGE ORTHOPEDIQUE ET TRAUMATOLOGIQUE

(30) Priority: 07.05.1998 IT PD980110
(43) Date of publication of application: 07.03.2001
(73) Proprietor: Martella, Pasquale, 35010 Vigodarzere (Padova) (IT)
(72) Inventor: Martella, Pasquale, 35010 Vigodarzere (Padova) (IT)
(74) Representative: Modiano, Guido, Dr.-Ing.
(86) International application number: PCT/IB1999/000801
(87) International publication number: WO 1999/056651

(56) References cited:
- FR-A- 2 727 304
- SU-A- 1 147 378
- US-A- 5 584 695

## Description

### TECHNICAL FIELD

The present invention relates to a synthesis device for orthopaedia and traumatology.

More particularly, the present invention relates to a device for the synthesis of a bone fracture after its reduction by means of a body which allows to fix the fractured region, keeping it in the correct position for the time required by the bone to strengthen.

### BACKGROUND ART

Various devices used to perform this operation are known.

Among these, the devices most frequently used are screws of the self-tapping or through type, possibly cooperating with plates which are arranged laterally to the bone in the region where the fracture is present.

All these devices are very troublesome to position and also have problems linked, among other issues, to the compactness of the bone in the fractured region.

More particularly, screws of the self-tapping type rely on a good grip of the thread, but this does not always occur.

Through screws associated with external plates are very bulky and must usually be removed when the bone has consolidated in the fracture region.

Substantially U-shaped brackets are also known which are made of materials having shape memory; their wings are inserted in two holes formed in the bone on opposite sides with respect to the fracture line.

These brackets, when heated, shorten in the intermediate region between the two ends inserted in the bone or the two ends bend toward each other, connecting the fracture.

However, these brackets have the drawback that they act only by traction in the external cortical region of the bone and in many cases do not allow to control the fracture region and keep it in the correct position.

US-A-5584695 discloses an apparatus for anchoring a prosthetic tooth 21 to a patient's jawbone 22 including a root screw 26, with a longitudinal bore 29, for implantation in the jawbone 22, and a temperature-sensitive shape-memory cylindrical coupling pin 34 for fixing a stump 35, with its own longitudinal bore 39 and mounting the prosthetic tooth 21, to the root screw 26. The coupling pin 34 extends into the aligned bores 29 and 39, and at temperatures below its transformation temperature range (TTR) the coupling pin 34 is thinner than the diameters of such bores 29 and 39 for easy insertion and extraction therefrom, while at temperatures above its TTR the coupling pin 34 expands inside the bores 29 and 39 to lock the mounting stump 35 to the root screw 26. In one specific embodiment (FIG. 2C), the cylindrical coupling pin 34 has a forked end portion 47 with two or more prongs 48 which open up at temperatures above the TTR for locking the coupling pin 34 into either or both bores 29 and 39.

FR-A-2727304 discloses a self-locking medullary canal bone implant having at least one section made from a metal alloy with a shape memory effect, situated at one or both ends or along its whole length. The implant is shaped for convenient positioning and its shape changes on heating or cooling so that it grips the inside of the bone and holds firmly in place.

### DISCLOSURE OF THE INVENTION

The aim of the present invention is to provide a synthesis device for orthopaedia and traumatology which can advantageously overcome the described problems.

A consequent primary object is to provide a device which can be easily inserted in the fracture region and is capable of keeping the fractured region correctly aligned.

Another object is to provide a synthesis device which, once inserted, can compact the fracture region.

Another object is to provide a synthesis device which has small dimensions, so that it can be left in the bone also after said bone has consolidated in the fracture region.

In accordance with the invention, there is provided a synthesis device for orthopaedia and traumatology as defined in the appended claims.

### BRIEF DESCRIPTION OF THE DRAWINGS

Further characteristics and advantages of the invention will become apparent from the description of some embodiments thereof, illustrated only by way of non-limitative example in the accompanying drawings, wherein:
Figure 1 is a partial sectional view of the device according to the invention;
Figures 2a, 2b and 2c are views of three types of the same device with a different number of sectors of the end parts;
Figure 3 is a view of the individual synthesis device installed to lock a region after reduction of the fracture;
Figure 4 is a view of the insertion of the device of Figure 3 by means of an auxiliary guiding tool;
Figure 5 is a view of the correct positioning of the device and of the extraction of the auxiliary guiding tool;
Figure 6 is a view of the activation of the device by means of an electric scalpel;
Figure 7 is a view of the arrangement of two devices in an extensive fracture;
Figure 8 is a view of a step of the insertion of the device with intramedullary placement;
Figure 9 is a view of the activation of the device of Figure 8;
Figure 10 is a view of the device used for Austin osteotomy of the first metatarsus;
Figure 11 is a view of two cylinders associated with a flat frame;
Figure 12 is a view of the connection of the device of Figure 11 for reducing a fracture;
Figure 13 is a front view of the deformation of the flat frame with shortening of the center distance between the two cylinders;
Figure 14 is a view of the curved shape assumed by the flat frame after heating.

### WAYS OF CARRYING OUT THE INVENTION

With reference to the above figures, the synthesis device according to the invention is composed of a cylindrical body, generally designated by the reference numeral 10, which has a median region 11 and two end regions 12 and 13.

In the illustrated embodiment, each one of the end regions 12 and 13 is divided into sectors, respectively 12a and 13a, which are divided along diametrical planes.

The entire cylinder is crossed by an axial hole designated by the reference numeral 14.

Figures 2a, 2b and 2c illustrate different shapes of the end regions, respectively divided into 4, 3 and 2 sectors; the axial hole 14 is always provided in each case.

The synthesis device is made of a Ni-Ti alloy with thermoelastic transformation and shape memory.

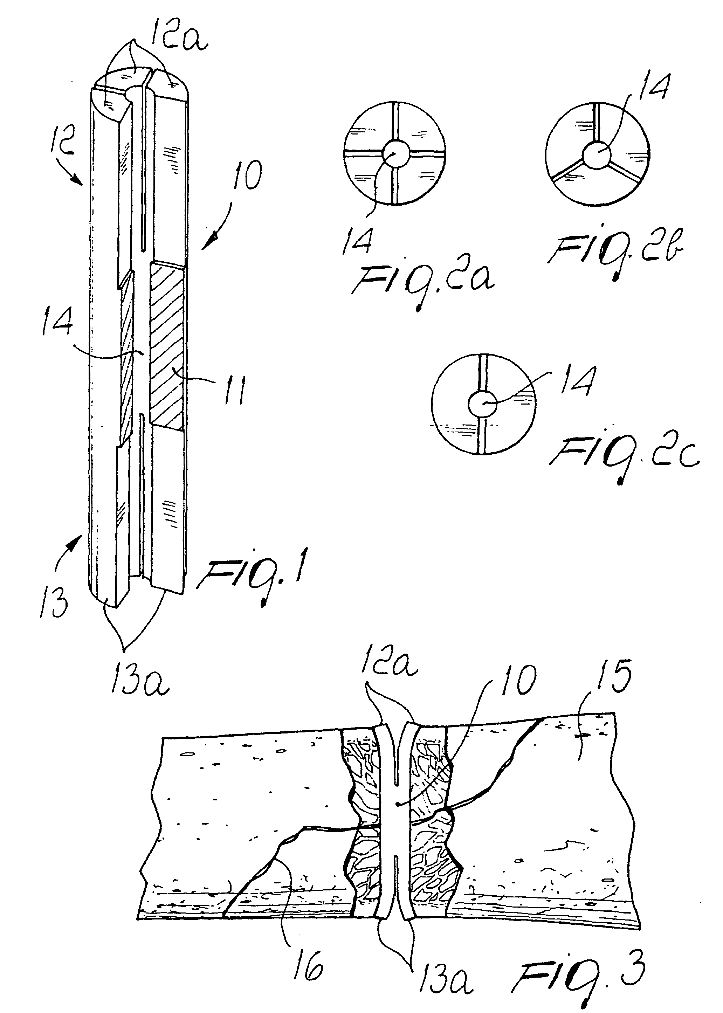

Substantially, the device is produced with a shape which is different from the one shown in Figure 1 and is given to said cylinder with a known technology.

The cylinder has a memory of the previous shape which is known as:
-- hot memory, when the split end begins to open out at 35°C, stops opening at 55°C and malleability occurs at 20°C;
-- cold memory, when the split end begins to open out at 20°C, stops opening at 35°C and malleability occurs at 10°C.

Due to the above-defined temperatures, the sectors of the split end return to the memorized shape, which is then maintained.

Figure 3 illustrates the device in position for compacting a fractured region of a bone 15, wherein the already-reduced fracture line is designated by the reference numeral 16.

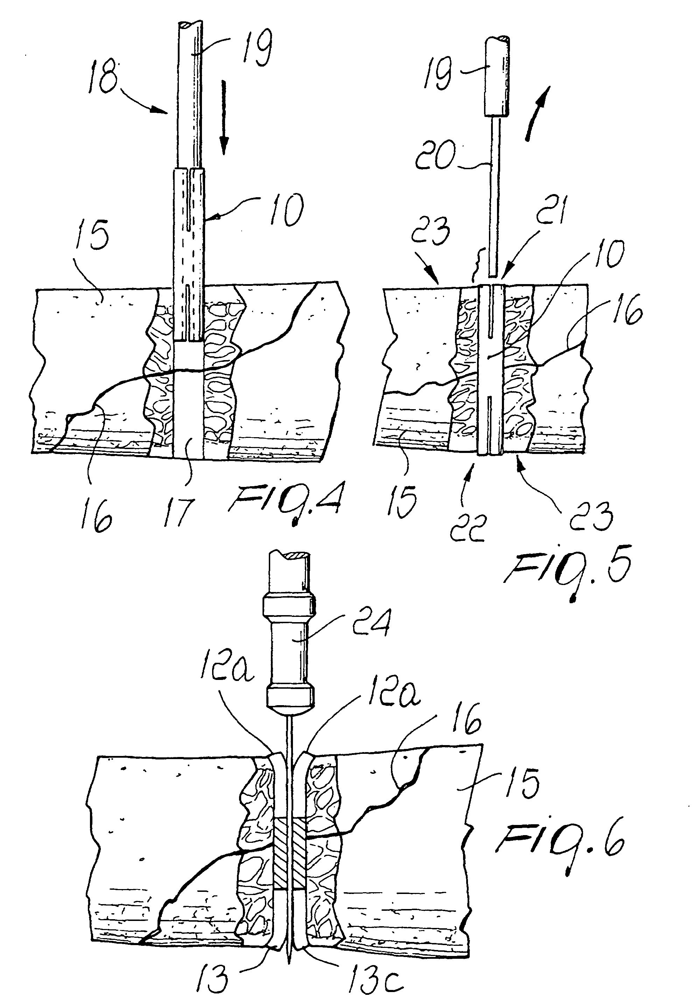

The operations for placing the synthesis device are shown in Figures 4, 5 and 6.

After reducing the fracture, whose line is again designated by the reference numeral 16, the bone being again designated by the reference numeral 15, a through hole 17 is formed and the device 10 is inserted by means of an auxiliary guiding tool 18 which is constituted by a handle body 19 which has, at its end, a needle 20 which has a slightly smaller diameter than the hole 14 that passes through the device 10.

With this auxiliary guiding tool it is possible to insert the device in the hole 17 as shown in Figure 5.

The length of the device is chosen so as to be slightly greater than the length of the bone in the fractured region, so that its ends, designated by the reference numerals 21 and 22, protrude by approximately 2 mm from the outer cortical region of the bone, designated by the reference numeral 23.

The end of an electric scalpel 24 is then inserted in the hole 14 as shown in Figure 6.

The heating produced by the electric scalpel causes the device to return to the memorized shape, opening out the sectors 12a and 13a with a particularly large spacing in the end regions.

In this way, in addition to maintaining the reduction of the fracture for the insertion of the device, compression of the two segments of the bone at the fracture line is also achieved.

Conveniently, the device has a diameter of approximately 3 mm, with an axial hole of 1 mm, but these dimensions, as well as the length, can differ according to requirements.

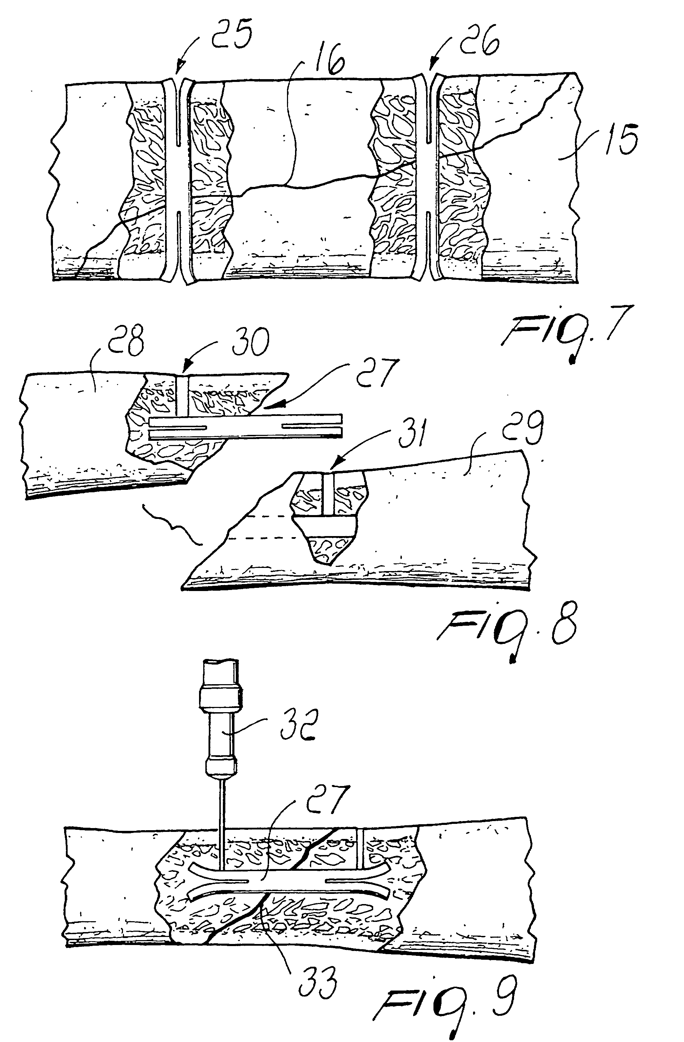

Figure 7 illustrates a reduction of an extensive fracture by means of two synthesis devices, now generally designated by the reference numerals 25 and 26, while the bone is again designated by the reference numeral 15 and the fracture line is again designated by the reference numeral 16.

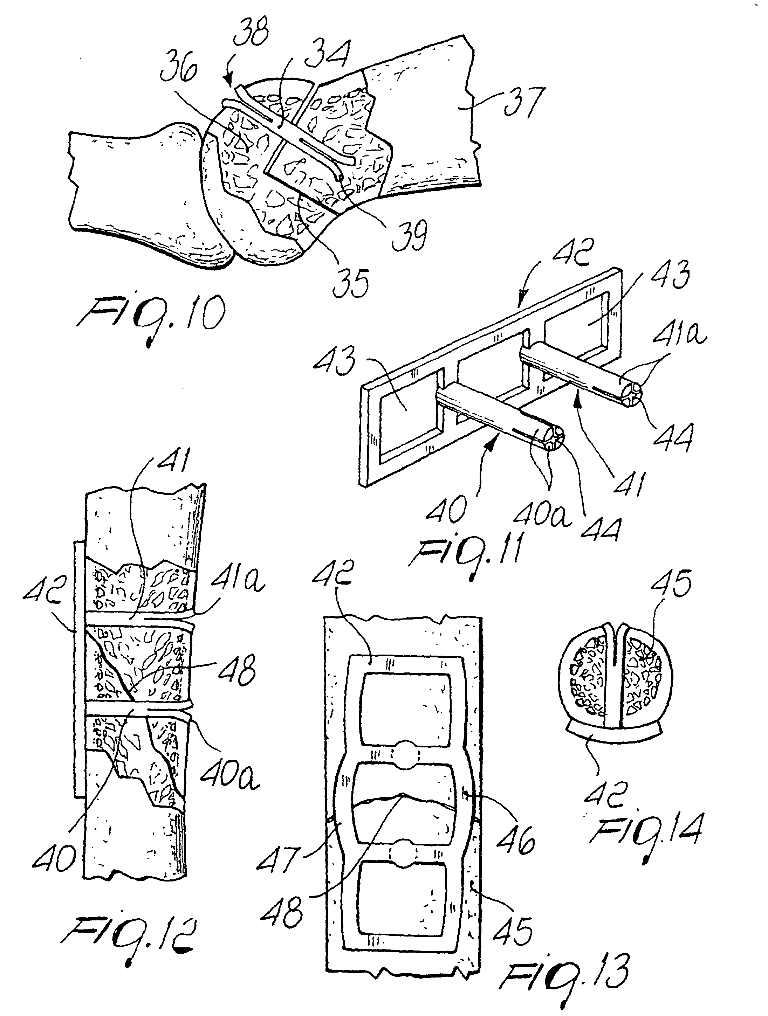

Figures 8 and 9 illustrate the synthesis device fitted in an intramedullary manner.

In this case, the device, now designated by the reference numeral 27, is first placed on one fractured bone segment, designated by the reference numeral 28, while the second segment, designated by the reference numeral 29, is displaced in order to allow the operation.

The second segment 29 is then moved into its correct position, so that the synthesis device 27 is in an intramedullary position.

Two holes, designated by the reference numerals 30 and 31 respectively, are formed beforehand in the two segments and are located at the two ends of the device 27.

After reducing the fracture as shown in Figure 9, the electric scalpel, now designated by the reference numeral 32, is inserted; said scalpel heats and causes the spacing of the end sectors of the synthesis device 27.

Also in this case, by way of said spacing the fracture line, now designated by the reference numeral 33, is compressed.

Figure 10 illustrates the use of the synthesis device, now designated by the reference numeral 34, for Austin osteotomy of the first metatarsus.

In this case, after producing a cut, with a known technique, along the broken line 35 and after correctly positioning the two bone parts 36 and 37, a blind hole is formed and the synthesis device 34 is inserted therein.

Heating by means of an electric scalpel allows the sectors, now designated by the reference numeral 38, to open out on the outer cortex and allows the sectors of the other end, now designated by the reference numeral 39, to open out in the spongy region of the head.

Figures 11, 12; 13 and 14 illustrate a synthesis device which constitutes a different embodiment of the inventive concept.

In this case, two cylinders, now designated by the reference numerals 40 and 41, are associated at right angles with a flat frame 42 which has perforated regions 43.

The two cylinders 40 and 41 have free ends which are again divided into sectors, now designated by the reference numerals 40a and 41 a, and there is again an axial hole designated by the reference numeral 44.

The entire unit is monolithic and again made of the same alloy having shape memory.

In this case, the memorized shape is such that heating, again performed by means of an electric scalpel, in addition to opening out the end sectors 40a and 41a of the cylinders 40 and 41, also bends the flat frame as shown in Figure 14, so as to adapt to the rounded shape of the bone, now designated by the reference numeral 45.

The flat frame again changes shape, curving the two connections 46 and 47 and thus tending to move the cylinders 40 and 41 mutually closer.

This produces the compression of the fracture line designated by the reference numeral 48.

From the description and the illustrations it is evident that the intended aim and all of the objects have been achieved.

The new synthesis device for orthopaedia and traumatology is very simple and easy to install.

Once the fracture or osteotomy has been reduced, the bone is prepared as if to place a screw and then the metal cylinder is inserted, with the suitable guiding tool as shown, in the hole prepared beforehand.

It is then sufficient to insert an electric scalpel in the axial hole of the cylinder to open out the sectors and/or deform the plate.

After the installation and opening out of the device, optimum and prolonged compression between the fragments is provided.

The compression force can vary between 3 and 5 kg depending on the diameter and length of the metal cylinder.

As mentioned, the device can be used as a means for intramedullary synthesis.

In this case, the sectors that open out grip the internal cortex.

The main recommendations for the use of the synthesis device are fractures and osteotomies of the metatarsi and metacarpi, arthrodeses of the interphalangeal articulations through an intramedullary pathway, arthrodeses of the first metatarsal-phalangeal joint, clavicular fractures through an intramedullary pathway, Austin osteotomy of the first metatarsus, etcetera.

The main indications for using the device provided with a flat frame are fractures of the distal diaphysis and epiphysis of the radium, ulnar fractures, clavicular fractures, etcetera.

The embodiments may obviously be different as convenient starting from the same inventive concept and always using a material with shape memory.

The disclosures in Italian Patent Application No. PD98A000110 from which this application claims priority are incorporated herein by reference.

## Claims

1. A synthesis device for orthopaedia and traumatology, comprising a cylinder (10,25,26,40,41) which is made of a Ni-Ti alloy having shape memory (hot, cold or superelastic), wherein at least one of the two ends (12,13) is split and can be opened out by heat, assuming a memorized shape and maintaining it after cooling, **characterized by** said cylinder (10) having a median region (11) which continues with two end regions (12,13) which are split because each one is divided into a plurality of sectors (12a,13a), and by an axial hole (14) being provided through said cylinder (10) and said axial hole (14) having dimensions sufficient for the insertion of a heating electric scalpel (24).

2. The device according to claim 1, wherein the Ni-Ti alloy with hot memory is **characterized in that** the opening out of the split end or ends (12,13) begins at approximately 35°C, ends at approximately 55°C and **in that** malleability occurs at approximately 20°C.

3. The device according to claim 1, wherein the Ni-Ti alloy with cold memory is **characterized in that** the opening out of the split end or ends (12,13) begins at approximately 20°C, ends at approximately 35°C and **in that** malleability occurs at approximately 10°C.

4. The synthesis device according to claim 1, **characterized in that** said cylinder having a chosen length which is sufficiently long such that when inserted in a through hole (17) traversing a fracture line (16,48) of a fractured region of a bone (15,45) said at least one of the two ends protrudes by a short extent from the outer cortical region (23) of the bone so that said opening-out by heating produces the compaction and coupling of the two fracture segments.

5. The synthesis device according to claim 1, **characterized in that** both ends (12,13) protrude by a short extent from the outer cortical region (23) of the bone so that said opening-out by heating produces the compaction and coupling of the two fracture segments.

6. A synthesis device for orthopaedia and traumatology, comprising at least two cylinders (40,41) with one end which is divided into sectors (40a,41a) and another end which is associated at right angles with a plate which is perforated so as to form a frame (42), the entire assembly being made of the same shape-memory alloy, said sectors of said cylinders opening out due to the heat and said frame, again due to the heat, shortening in the region. between the cylinders in order to compact the fracture region and bending as a whole toward the cylinders in order to assume a shape which adapts to the shape of the bone to which it is applied.

7. The synthesis device for orthopaedia and traumatology according to claims 1 and/or 4, **characterized in that** said ends of the cylinder are divided into 4 sectors (12a, 13a, 40a, 41a).

## Patentansprüche

1. Synthesevorrichtung für Orthopädie und Traumatologie
- mit einem Zylinder (10, 25, 26,40,41), der aus einer Ni-Ti-Legierung hergestellt ist, die ein Formgedächtnis hat (heiß, kalt oder superelastisch),
- wobei wenigstens eines der beiden Enden (12, 13) geteilt ist und durch Wärme nach außen geöffnet werden kann,
- wobei es eine gespeicherte Form annimmt und diese nach dem Abkühlen beibehält,
**dadurch gekennzeichnet,**
- **dass** der Zylinder (10) einen Mittelbereich (11) hat, der sich in zwei Endbereichen (12, 13) fortsetzt, die geteilt sind, indem jeder von ihnen in eine Vielzahl von Sektoren (12a, 13a) unterteilt ist, und
- **dass** ein durch den Zylinder (10) hindurchgehendes axiales Loch (14) mit Abmessungen vorgesehen ist, die für das Einführen eines elektrischen Heizskalpells (24) ausreichen.

2. Vorrichtung nach Anspruch 1, bei welcher sich die Ni-Ti-Legierung mit Heiß-Gedächtnis dadurch auszeichnet, dass das nach außen Öffnen des geteilten Endes oder der geteilten Enden (12, 13) bei etwa 35°C beginnt und bei etwa 55°C endet und dass die Verformbarkeit bei etwa 20°C eintritt.

3. Vorrichtung nach Anspruch 1, bei welcher sich die Ni-Ti-Legierung mit Kalt-Gedächtnis dadurch auszeichnet, dass das nach außen Öffnen des geteilten Endes oder der geteilten Enden (12, 13) bei etwa 20°C beginnt und bei etwa 35°C endet und dass die Verformbarkeit bei etwa 10°C eintritt.

4. Synthesevorrichtung nach Anspruch 1, **dadurch gekennzeichnet, dass** der Zylinder eine ausgewählte Länge hat, die ausreichend lang ist, so dass, wenn er in ein Durchgangsloch (17), das eine Bruchlinie (16, 48) eines Frakturbereichs eines Knochens (15, 45) durchquert, das wenigstens eine der beiden Enden mit einer kurzen Erstreckung von dem äußeren Kortikalbereich (23) des Knochens aus so vorsteht, dass das nach außen Öffnen durch Erhitzen die Verhakung und Koppelung der beiden Bruchsegmente erzeugt.

5. Synthesevorrichtung nach Anspruch 1, **dadurch gekennzeichnet, dass** die beiden Enden (12, 13) mit einer kurzen Erstreckung von dem äußeren Kortikalbereich (23) des Knochens so vorstehen, dass das nach außen Öffnen durch Erhitzen die Verhakung und Koppelung der beiden Bruchsegmente erzeugt.

6. Synthesevorrichtung für Orthopädie und Traumatologie, welche wenigstens zwei Zylinder (40, 41) aufweist, deren eines Ende in Sektoren (40a, 41a) und deren anderem Ende im rechten Winkel eine Platte zugeordnet ist, die zur Bildung eines Rahmens (42) perforiert ist, wobei die gesamte Anordnung aus der gleichen Formgedächtnislegierung hergestellt ist, die Sektoren der Zylinder sich aufgrund der Wärme nach außen öffnen und der Rahmen wiederum aufgrund von Wärme sich in dem Bereich zwischen den Zylindern verkürzt, um den Bruchbereich zu verhaken, und sich als Ganzes zu den Zylindern hin biegt, um eine Form anzunehmen, die der Form des Knochens angepasst ist, an dem er angebracht ist.

7. Synthesevorrichtung für Orthopädie und Traumatologie nach Anspruch 1 und/oder 4, **dadurch gekennzeichnet, dass** die Enden des Zylinders in vier Sektoren (12a, 13a, 40a, 41a) aufgeteilt sind.

## Revendications

1. Dispositif de synthèse pour l'orthopédie et la traumatologie, comprenant un cylindre (10, 25, 26, 40, 41) qui est réalisé en un alliage Ni-Ti à mémoire de forme (à chaud, à froid ou super-élastique), dans lequel au moins l'une des deux extrémités (12, 13) est fendue et peut s'ouvrir par la chaleur, en prenant une forme mémorisée et en la conservant après refroidissement,
**caractérisé en ce que** ledit cylindre (10) a une région médiane (11) qui se prolonge avec deux régions d'extrémité (12, 13) qui sont fendues puisque chacune est divisée en une pluralité de secteurs (12a, 13a), et **en ce qu'**un trou axial (14) est prévu à travers ledit cylindre (10) et ledit trou axial (14) ayant des dimensions suffisantes pour l'insertion d'un scalpel électrique chauffant (24).

2. Dispositif selon la revendication 1, dans lequel l'alliage Ni-Ti à mémoire à chaud est **caractérisé en ce que** l'ouverture de l'extrémité fendue ou des extrémités fendues (12, 13) commence à approximativement 35°C, se termine à approximativement 55°C et **en ce que** la malléabilité se produit à approximativement 20°C.

3. Dispositif selon la revendication 1, dans lequel l'alliage Ni-Ti à mémoire à froid est **caractérisé en ce que** l'ouverture de l'extrémité fendue ou des extrémités fendues (12, 13) commence à approximativement 20°C, se termine à approximativement 35°C et **en ce que** la malléabilité se produit à approximativement 10°C.

4. Dispositif de synthèse selon la revendication 1,
**caractérisé en ce que** ledit cylindre a une longueur choisie qui est suffisamment longue de telle sorte que, quand il est inséré dans un trou débouchant (17) traversant une ligne de fracture (16, 48) d'une région fracturée d'un os (15, 45), ladite extrémité fait saillie d'une courte distance de la région corticale externe (23) de l'os, de sorte que ladite ouverture, en chauffant, produit le compactage et l'assemblage des deux segments fracturés.

5. Dispositif de synthèse selon la revendication 1,
**caractérisé en ce que** les deux extrémités (12, 13) font saillie d'une courte distance de la région corticale externe (23) de l'os, de sorte que ladite ouverture, en chauffant, produit le compactage et l'assemblage des deux segments fracturés.

6. Dispositif de synthèse pour l'orthopédie et la traumatologie, comprenant au moins deux cylindres (40, 41) avec une extrémité qui est divisée en secteurs (40a, 41 a) et une autre extrémité qui est associée à angle droit à une plaque qui est perforée de façon à former un cadre (42), l'ensemble total étant réalisé avec le même alliage à mémoire de forme, lesdits. secteurs desdits cylindres s'ouvrant à cause de la chaleur et ledit cadre, de nouveau à cause de la chaleur, se raccourcissant dans la région entre les cylindres de façon à compacter la région fracturée et à se courber dans sa totalité vers les cylindres de façon à prendre une forme qui s'adapte à la forme de l'os auquel il est appliqué.

7. Dispositif de synthèse pour l'orthopédie et la traumatologie selon la revendication 1 et/ou la revendication 4,
**caractérisé en ce que** lesdites extrémités du cylindre sont divisées en quatre secteurs (12a, 13a, 40a, 41a).
